# EUROPEAN PATENT APPLICATION

(11) **EP 4 772 159 A1**
(43) Date of publication of application: **08.07.2026**
(21) Application number: 24859106.7
(22) Date of filing: 18.06.2024
(51) Int. Cl.: A61K 6/836, A61K 6/15, A61K 6/887

(54) **GLASS COMPOSITION FOR DENTAL USE AND COMPOSITION FOR DENTAL USE**

(30) Priority: 31.08.2023 JP 2023140970
(71) Applicant: GC R&D Corporation, Tokyo 174-8585 (JP)
(72) Inventor: FUNAYAMA, Naoya, Tokyo 174-8585 (JP); MIYAKE, Takahiro, Tokyo 174-8585 (JP); KASAI, Yuki, Tokyo 174-8585 (JP); MINAMISAWA, Hiroto, Tokyo 174-8585 (JP)
(74) Representative: Müller-Boré & Partner Patentanwälte PartG mbB
(86) International application number: PCT/JP2024/022123
(87) International publication number: WO 2025/047060

(57) **Abstract**

A dental glass composition contains silicon (Si) and cesium (Cs). The dental glass composition is substantially free from sodium (Na), potassium (K), and one or more alkaline earth metals.

## Description

### TECHNICAL FIELD

The present invention relates to dental glass compositions and dental compositions.

### BACKGROUND OF THE INVENTION

In the field of dentistry, a dental glass composition including barium has been known as a filler (filling material) to be blended in a dental curable composition. Since glass including barium (barium glass) has high radiopacity, the barium glass has been widely used as a raw material of a filler for a dental material that requires radiopacity (see, for example, Patent Document 1).

### RELATED-ART DOCUMENT

### Patent Document

Patent Document 1: Japanese Patent No. 6346086

### SUMMARY OF THE INVENTION

### PROBLEMS TO BE SOLVED BY THE INVENTION

However, the existing glass including barium includes a polyvalent metal. In a dental composition in which an acidic substance, such as an acidic group-containing polymerizable monomer or the like is present in combination with the glass, therefore, the polyvalent metal and the acidic group cause adsorption or a reaction, thereby causing problems in storage stability, such as changes in properties of a paste, a reduction in adhesive strength, and the like.

The object of the present invention is to provide a dental glass composition that achieves both high radiopacity of a dental composition to be obtained and storage stability in the presence of an acidic substance.

### MEANS FOR SOLVING THE PROBLEMS

A dental glass composition according to one aspect of the present invention includes silicon (Si) and cesium (Cs), and is substantially free from sodium (Na), potassium (K), and one or more alkaline earth metals.

### EFFECTS OF THE INVENTION

According to one aspect of the present invention, there can be provided a dental glass composition that can achieve both high radiopacity of a dental composition to be obtained and storage stability in the presence of an acidic substance.

### DETAILED DESCRIPTION OF THE INVENTION

Embodiments of the present invention will be described in detail hereinafter.

### <Dental glass composition>

The dental glass composition according to the present embodiment includes silicon (Si) and cesium (Cs).

The silicon (Si) is present as an oxide of silicon (Si) (silicon oxide (SiO₂)) in the dental glass composition.

The amount of silicon (Si) determined by oxide conversion is not particularly limited. For example, the amount of silicon (Si) determined by oxide conversion is preferably 10 percent by mass or greater and 80 percent by mass or less, more preferably 20 percent by mass or greater and 70 percent by mass or less, and yet more preferably 30 percent by mass or greater and 60 percent by mass or less in the dental glass composition.

When the amount of silicon (Si) determined by oxide conversion is 10 percent by mass or greater, a reduction in chemical resistance of glass and devitrification can be avoided. When the amount of silicon (Si) determined by oxide conversion is 80 percent by mass or less, the glass melt can be inhibited from becoming hard so that the viscosity of the dental glass composition can be easily adjusted, leading to a reduction in production cost of the dental glass composition.

Cesium (Cs) is present as an oxide of cesium (Cs) (cesium oxide (Cs₂O)) in the dental glass composition. Cesium (Cs) is an alkali metal having the atomic number or electron number of 55, and exhibits high radiopacity in glass.

In the present specification, the term "radiopacity" refers to a property such that a density of an X-ray image changes depending on the X-ray absorption amount of an object of interest, such as biological tissues, a material, or the like, thereby visualizing or distinguishing the object of interest.

In dental treatments, it is necessary to clearly visualize the treated site after the procedure using a dental material, such as a dental filler or the like, and therefore such radiopacity is required for dental materials. Since a resin matrix used in a dental material is an organic material, the resin matrix does not have radiopacity, and a filler formed of an inorganic material, such as glass, is responsible for imparting radiopacity to the dental material. Accordingly, the radiopacity is an important property for dental glass.

The amount of cesium (Cs) is not particularly limited. For example, the amount of cesium (Cs) determined by oxide conversion is 5 percent by mass or greater and 55 percent by mass or less, preferably 10 percent by mass or greater and 45 percent by mass or less, and more preferably 20 percent by mass or greater and 35 percent by mass or less in the dental glass composition.

When the amount of cesium (Cs) determined by oxide conversion is 5 percent by mass or greater, radiopacity of the dental glass composition can be enhanced. When the amount of cesium (Cs) determined by oxide conversion is 55 percent by mass or less, a sufficient amount of silicon (Si) in the dental glass composition can be secured, and a reduction in chemical resistance can be inhibited.

The dental glass composition of the present embodiment may further include boron (B), aluminum (Al), zirconium (Zr), or fluorine (F).

Boron (B) can be present as an oxide of boron (B) (boron oxide (B₂O₃)) in the dental glass composition.

In the case where boron (B) is included, the amount of boron (B) is not particularly limited. The amount of boron (B) determined by oxide conversion is preferably 1 percent by mass or greater and 45 percent by mass or less, more preferably 5 percent by mass or greater and 35 percent by mass or less, and yet more preferably 10 percent by mass or greater and 25 percent by mass or less in the dental glass composition.

When the amount of boron (B) determined by oxide conversion is 1 percent by mass or greater, chemical resistance of the dental glass composition can be improved, and a melting point of the glass melt is lowered so that the dental glass composition can be easily produced. When the amount of boron (B) determined by oxide conversion is 45 percent by mass or less, the phase separation of the glass in the dental glass composition can be inhibited, and deviation of the composition due to evaporation can be inhibited.

Aluminum (Al) can be present as an oxide of aluminum (Al) (aluminum oxide (Al₂O₃)) in the dental glass composition.

In the case aluminum (Al) is included, the amount of aluminum (Al) is not particularly limited. For example, the amount of aluminum (Al) determined by oxide conversion is preferably 0.1 percent by mass or greater and 35 percent by mass or less, more preferably 0.5 percent by mass or greater and 25 percent by mass or less, and yet more preferably 1 percent by mass or greater and 15 percent by mass or less in the dental glass composition.

When the amount of aluminum (Al) determined by oxide conversion is 0.1 percent by mass or greater, chemical resistance of the dental glass composition can be improved, and devitrification of glass can be inhibited. When the amount of aluminum (Al) determined by oxide conversion is 35 percent by mass or less, a melting point of a glass melt of the dental glass composition is lowered so that the dental glass composition can be easily produced.

Zirconium (Zr) can be present as an oxide of zirconium (Zr) (zirconium oxide (ZrO₂)) in the dental glass composition.

In the case where zirconium (Zr) is included, the amount of zirconium (Zr) is not particularly limited. The amount of zirconium (Zr) determined by oxide conversion is preferably 0.1 percent by mass or greater and 20 percent by mass or less, more preferably 0.5 percent by mass or greater and 15 percent by mass or less, and yet more preferably 1 percent by mass or greater and 10 percent by mass or less in the dental glass composition.

When the amount of zirconium (Zr) determined by oxide conversion is 0.1 percent by mass or greater, chemical resistance of glass can be improved, and radiopacity of glass to be obtained can be improved. When the amount of zirconium (Zr) determined by oxide conversion is 20 percent by mass or less, a melting point of the glass melt of the dental glass composition is lowered so that the dental glass composition can be easily produced.

Fluorine (F) can be present as fluoride or a fluoride salt in the dental glass composition.

In the case where fluorine (F) is included, the amount of fluorine (F) is not particularly limited. The amount of fluoride (F) as a single substance is preferably 0.1 percent by mass or greater and 10 percent by mass or less, more preferably 0.5 percent by mass or greater and 8 percent by mass or less, and yet more preferably 1 percent by mass or greater and 5 percent by mass or less in the dental glass composition.

When the amount of fluorine (F) is 0.1 percent by mass or greater, the viscosity of the dental glass composition becomes low, productivity of the dental glass composition can be improved, and a refractive index of the dental glass composition can be adjusted. When the amount of fluorine (F) is 10 percent by mass or less, devitrification of glass can be inhibited, and deviation of the composition due to evaporation can be inhibited.

The dental glass composition according to the present embodiment is substantially free from sodium (Na), potassium (K), and one or more alkaline earth metals.

In the present specification, the phrase "substantially free from" means that sodium (Na), potassium (K), and one or more alkaline earth metals are not intentionally included, but substances that are included inevitably (inevitable impurities) are allowed to be included.

Sodium (Na) is an alkali metal having an atomic number or an electron number of 11, and is an element that easily reacts with an acidic group, such as a phosphoric acid group, or the like. Moreover, potassium (K) is an alkali metal having an atomic number or an electron number of 19, and is an element that easily reacts with an acidic group, such as a phosphoric acid group, or the like.

The one or more alkaline earth metals are at least one element selected from the group consisting of Be (beryllium), magnesium (Mg), calcium (Ca), strontium (Sr), and barium (Ba), and any of the above elements is an element that easily reacts with an acidic group, such as a phosphoric acid group, or the like.

The allowable amount of sodium (Na) in the dental glass composition, which is determined by oxide conversion, is 1 percent by mass or less, preferably 0.8 percent by mass or less, and more preferably 0.5 percent by mass or less. Sodium (Na) that is allowed to be included in the dental glass composition is present as sodium oxide (Na₂O) in the dental glass composition.

When the amount of sodium (Na) determined by oxide conversion is greater than 1 percent by mass, sodium (Na) reacts with an acidic group, such as a phosphoric acid group or the like, causing a defect in storage stability in a paste. Therefore, the dental glass composition is preferably substantially free from sodium (Na).

In the present specification, the term "defect in storage stability" means that properties of a paste obtained by mixing the dental glass composition and an acidic substance (e.g., an acidic group-containing polymerizable monomer) change.

The allowable amount of potassium (K) in the dental glass composition, which is determined by oxide conversion, is 1 percent by mass or less, and preferably 0.8 percent by mass or less, and more preferably 0.5 percent by mass or less. Potassium (K) that is allowed to be included in the dental glass composition is present as potassium oxide (K₂O) in the dental glass composition.

When the amount of potassium (K) determined by oxide conversion is greater than 1 percent by mass, potassium (K) reacts with an acidic group (phosphoric acid group) so that a defect in storage stability is likely to occur in the paste. Therefore, the dental glass composition is preferably substantially free from potassium (K).

The allowable amount of the one or more alkaline earth metals in the dental glass composition is not particularly limited. For example, the allowable amount of the one or more alkaline earth metals determined by oxide conversion is 1 percent by mass or less, preferably 0.8 percent by mass or less, and more preferably 0.5 percent by mass or less.

The one or more alkaline earth metals that are allowed to be included in the dental glass composition are present as one or more alkaline earth metal oxides, such as beryllium oxide (BeO), magnesium oxide (MgO), calcium oxide (CaO), strontium oxide (SrO), or the like, in the dental glass composition.

When the amount of the one or more alkaline earth metals determined by oxide conversion is greater than 1 percent by mass, the alkaline earth metal reacts with an acidic group, such as a phosphoric acid group or the like so that a defect in storage stability is likely to occur in the paste. Therefore, the dental glass composition is preferably substantially free from the one or more alkaline earth metals.

The one or more alkaline earth metals from which the dental glass composition is free in the dental glass composition are, in particular, calcium (Ca), strontium (Sr), and barium (Ba). Among alkaline earth metals, calcium (Ca), strontium (Sr), and barium (Ba) easily react with acidic groups, such as a phosphoric acid or the like. Since the dental glass composition is substantially free from the above alkaline earth metals, occurrences of defect in storage stability in the paste can be inhibited.

The dental glass composition of the present embodiment may further include other elements, as long as such elements do not adversely affect the object of the present invention. Examples of other elements include zinc (Zn), tin (Sn), lanthanum (La), gadolinium (Gd), rubidium (Rb), and the like, from the viewpoints of improvements in radiopacity and chemical resistance, the viewpoint of inhibition of devitrification, the viewpoint of easy production of the dental glass composition, or the like. The other elements can be present as oxides in the dental glass composition. The amount of the other elements is not particularly limited, and any mass of the other elements is included in the dental glass composition.

Zinc (Zn) can be present as an oxide of zinc (Zn) (zinc oxide (ZnO)) in the dental glass composition.

In the case where zinc (Zn) is included, the amount of zinc (Zn) is not particularly limited. The amount of zinc (Zn) determined by oxide conversion is preferably 0.1 percent by mass or greater and 20 percent by mass or less, more preferably 0.5 percent by mass or greater and 15 percent by mass or less, and yet more preferably 1 percent by mass or greater and 10 percent by mass or less in the dental glass composition.

When the amount of zinc (Zn) determined by oxide conversion is 0.1 percent by mass or greater, chemical resistance of the dental glass composition can be improved, and devitrification of glass can be inhibited. When the amount of zinc (Zn) determined by oxide conversion is 20 percent by mass or less, devitrification can be inhibited without lowering chemical resistance of glass to be obtained, and the dental glass composition can be easily produced.

Lanthanum (La) can be present as an oxide of lanthanum (La) (lanthanum oxide (La₂O₃)) in the dental glass composition.

In the case where lanthanum (La) is included, the amount of lanthanum (La) is not particularly limited. The amount of lanthanum (La) determined by oxide conversion is preferably 1 percent by mass or greater and 40 percent by mass or less, more preferably 5 percent by mass or greater and 35 percent by mass or less, and yet more preferably 10 percent by mass or greater and 30 percent by mass or less in the dental glass composition.

When the amount of lanthanum (La) determined by oxide conversion is 1 percent by mass or greater, radiopacity of glass to be obtained can be improved. When the amount of lanthanum (La) determined by oxide conversion is 40 percent by mass or less, meltability of glass can be improved so that the dental glass composition can be easily produced.

Gadolinium (Gd) can be present as an oxide of gadolinium (Gd) (gadolinium oxide (Gd₂O₃)) in the dental glass composition.

In the case where gadolinium (Gd) is included, the amount of gadolinium (Gd) determined by oxide conversion is preferably 1 percent by mass or greater and 30 percent by mass or less, more preferably 3 percent by mass or greater and 25 percent by mass or less, and yet more preferably 5 percent by mass or greater and 20 percent by mass or less in the dental glass composition.

When the amount of gadolinium (Gd) determined by oxide conversion is 1 percent by mass or greater, radiopacity of glass to be obtained can be improved. When the amount of gadolinium (Gd) is 30 percent by mass or less, meltability of glass can be improved so that the dental glass composition can be easily produced.

Rubidium (Rb) can be present as an oxide of rubidium (Rb) (rubidium oxide (Rb₂O)) in the dental glass composition.

The amount of rubidium (Rb) is not particularly limited. For example, the amount of rubidium (Rb) determined by oxide conversion is 3 percent by mass or greater and 30 percent by mass or less, preferably 5 percent by mass or greater and 25 percent by mass or less, and more preferably 8 percent by mass or greater and 20 percent by mass or less in the dental glass composition.

When the amount of rubidium (Rb) determined by oxide conversion is 3 percent by mass or greater, radiopacity of the dental glass composition can be enhanced, and the melting point of the glass melt of the dental glass composition is lowered so that the dental glass composition can be easily produced. When the amount of rubidium (Rb) determined by oxide conversion is 30 percent by mass or less, a reduction in chemical resistance can be inhibited.

The dental glass composition of the present embodiment may include other inevitable impurities. Examples of the inevitable impurities include nickel (Ni) and the like.

The form of the dental glass composition is not particularly limited, and may be, for example, a powdery form or powder, a plate form, a columnar form, a thread form, or a fiber form. When the form of the dental glass composition is a powdery form, blending and mixing of the dental glass composition with constituent components of a dental composition become easy, and mechanical strength of the dental composition can be increased. When the form of the dental glass composition is a plate form or a columnar form, mechanical strength or transparency of a dental composition obtained by applying the dental glass composition to a dental material can be improved.

When the form of the dental glass composition is a powdery form, the particle size of the dental glass composition is not particularly limited. The median diameter of the dental glass composition is preferably 0.01 µm or greater and 20 µm or less, more preferably 0.02 µm or greater and 10 µm or less, and yet more preferably 0.1 µm or greater and 1 µm or less. The particle size means an average particle diameter defined by a median diameter.

When the particle size of the dental glass composition is 0.01 µm or greater, operability of the dental glass composition can be improved in the case where the dental glass composition is used as a powder. When the particle size of the dental glass composition is 20 µm or less, mechanical strength of a dental composition obtained when the dental glass composition is applied to a dental material can be improved.

The dental glass composition of the present embodiment can make the radiopacity of glass containing the dental glass composition 300% or greater, preferably 400% or greater, and more preferably 450% or greater compared to aluminum. In the present specification, the radiopacity refers to radiopacity measured by a radio-opacity test method of dental materials in accordance with ISO 4049:2019.

Since the radiopacity of the glass containing the dental glass composition is 300% or greater compared to aluminum in the present embodiment, a dental glass composition exhibiting favorable radiopacity can be provided. The upper limit of the radiopacity is not particularly limited, but is preferably 1,000% or less, more preferably 900% or less, and yet more preferably 800% or less, compared to aluminum, as an operable range.

As described above, the dental glass composition of the present embodiment includes silicon (Si) and cesium (Cs), and is substantially free from sodium (Na), potassium (K), and one or more alkaline earth metals, and therefore a dental composition obtained when the dental glass composition is applied to a dental material can achieve both high radiopacity and storage stability in the presence of an acidic substance.

In addition, as described above, in the dental glass composition of the present embodiment, the one or more alkaline earth metals from which the dental glass composition is free are calcium (Ca), strontium (Sr), and barium (Ba). Therefore, a defect in storage stability in a paste that includes the dental glass composition can be inhibited.

### <Dental composition>

The dental composition according to the present embodiment includes the above-described dental glass composition of the present embodiment and an acidic group-containing polymerizable monomer.

The acidic group-containing polymerizable monomer has a function of improving adhesion to dental tissues, ceramics serving as dental restorative materials, such as zirconia, alumina, and the like, and alloys including noble metals. The acidic group-containing polymerizable monomer is not particularly limited. Examples of the acidic group-containing polymerizable monomer include acid functional group-containing (meth)acrylate.

The acid functional group-containing (meth)acrylate monomer is not particularly limited. For example, the acid functional group-containing (meth)acrylate monomer is a (meth)acrylate monomer including an acid functional group, such as a phosphoric acid group, a pyrophosphoric acid group, a thiophosphoric acid group, a carboxy group, an acid anhydride residue, a sulfonic acid group, a phosphonic acid group, or the like. The acid functional group-containing (meth)acrylate may include two or more acid functional groups.

Examples of the phosphoric acid group-containing (meth)acrylate include 2-(meth)acryloyloxyethyl dihydrogen phosphate, bis[2-(meth)acryloyloxyethyl] hydrogen phosphate, 2-(meth)acryloyloxyethylphenyl hydrogen phosphate, 6-(meth)acryloyloxyhexyl dihydrogen phosphate, 6-(meth)acryloyloxyhexylphenyl hydrogen phosphate, 10-(meth)acryloyloxydecyl dihydrogen phosphate, 1,3-di(meth)acryloylpropane-2-dihydrogen phosphate, 1,3-di(meth)acryloylpropane-2-phenylhydrogen phosphate, bis[5-{2-(meth)acryloyloxyethoxycarbonyl}heptyl]hydrogen phosphate, and the like.

Examples of the pyrophosphoric acid group-containing (meth)acrylate include bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, bis[10-(meth)acryloyloxydecyl] pyrophosphate, and the like.

Examples of the thiophosphoric acid group-containing (meth)acrylate include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 13-(meth)acryloyloxytridecyl dihydrogen thiophosphate, 14-(meth)acryloyloxytetradecyl dihydrogen thiophosphate, 15-(meth)acryloyloxypentadecyl dihydrogen thiophosphate, 16-(meth)acryloyloxyhexadecyl dihydrogen thiophosphate, 17-(meth)acryloyloxyheptadecyl dihydrogen thiophosphate, 18-(meth)acryloyloxyoctadecyl dihydrogen thiophosphate, 19-(meth)acryloyloxynonadecyl dihydrogen thiophosphate, 20-(meth)acryloyloxyicosyl dihydrogen thiophosphate, and the like.

Examples of the carboxy group-containing (meth)acrylate include 2-methacryloyloxyethylsuccinic acid, 4-(meth)acryloyloxyethyl trimellitic acid, 4-(meth)acryloyloxydecyltrimellitic acid, 11-(meth)acryloyloxy-1,1-undecanedicarboxylic acid, 1,4-di(meth)acryloyloxypyromellitic acid, 2-(meth)acryloyloxyethylmaleic acid, 2-(meth)acryloyloxyethylphthalic acid, 2-(meth)acryloyloxyethylhexahydrophthalic acid, and the like.

Examples of the acid anhydride residue-containing (meth)acrylate include 4-(meth)acryloyloxyethyltrimellitic anhydride, 4-(meth)acryloyloxydecyltrimellitic anhydride, and the like.

Examples of the sulfonic acid group-containing (meth)acrylate include 2-(meth)acrylamide-2-methylpropanesulfonic acid, styrenesulfonic acid, 2-sulfoethyl (meth)acrylate, and the like.

Examples of the phosphonic acid group-containing (meth)acrylate include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phophonoacetate, and the like.

The above acid functional group-containing (meth)acrylate monomers may be used alone or in combination.

In view of solubility of a smear layer, which is on a tooth surface, to the dental composition and demineralization of dental tissues, particularly adhesion of the dental composition to enamels, the acid functional group-containing (meth)acrylate is preferably a phosphoric acid group, thiophosphoric acid group, carboxy group, or acid anhydride residue-containing (meth)acrylate. Among the above (meth)acrylate monomers, phosphoric acid group-containing (meth)acrylate is more preferable.

Further, among the above(meth)acrylate monomers, 10-methacryloyloxydecyl dihydrogen phosphate (MDP), 10-methacryloyloxydecyl dihydrogen thiophosphate (MDTP), 4-methacryloyloxyethyltrimellitic anhydride (4-META), or the like is preferable, and MDP is more preferable in view of an improvement in adhesion of the dental composition.

The amount of the acidic group-containing polymerizable monomer in the dental composition is not particularly limited. For example, the amount of the acidic group-containing polymerizable monomer can be set to 0.1 percent by mass or greater and 20 percent by mass or less, preferably 1 percent by mass or greater and 15 percent by mass or less, and more preferably 3 percent by mass or greater and 10 percent by mass or less.

When the amount of the acidic group-containing polymerizable monomer in the dental composition is 0.1 percent by mass or greater, adhesion of the dental composition to dental tissues is improved. When the amount of the acidic group-containing polymerizable monomer in the dental composition is 20 percent by mass or less, curability of the dental composition is improved.

The dental composition of the present embodiment may further include other components, as long as the object of the present invention is not impaired. Examples of the other components included in the dental composition include a polymerizable monomer having no acid functional group, a polymerization initiator, a polymerization inhibitor, a filler, a solvent, and the like. Examples of the polymerization initiator include a chemical polymerization initiator and a photopolymerization initiator.

The polymerizable monomer having no acid functional group is not particularly limited, and examples of the polymerizable monomer having no acid functional group include polyfunctional (meth)acrylate compounds. Among the polyfunctional (meth)acrylate compounds, a bifunctional (meth)acrylate compound is preferable. Examples of the bifunctional (meth)acrylate compound include ethoxylated bisphenol A dimethacrylate, 2,2-bis[4-[2-hydroxy-3-(methacryloyloxy)propyloxy]phenyl]propane, neopentyl glycol dimethacrylate, di-2-methacryloyloxyethyl 2,2,4-trimethylhexamethylene dicarbamate (UDMA), triethylene glycol dimethacrylate (TEGDMA), and the like. The above bifunctional (meth)acrylate compounds may be used alone or in combination.

The amount of the polymerizable monomer having no acid functional group in the dental composition is not particularly limited. For example, the amount of the polymerizable monomer having no acid functional group can be set to 1 percent by mass or greater and 40 percent by mass or less, preferably 3 percent by mass or greater and 35 percent by mass or less, and more preferably 5 percent by mass or greater and 33 percent by mass or less in the dental composition.

When the amount of the polymerizable monomer having no acid functional group in the dental composition is 1 percent by mass or greater, adhesion of the dental composition can be improved. When the amount of the polymerizable monomer having no acid functional group in the dental composition is 40 percent by mass or less, acid reactivity of the dental glass composition included in the dental composition is lowered, and therefore a defect in storage stability in the paste can be inhibited.

The polymerization initiator is not particularly limited. Examples of the polymerization initiator include a chemical polymerization initiator and a photopolymerization initiator.

The chemical polymerization initiator is not particularly limited. For example, a thiourea derivative, a vanadium compound, a tertiary amine, or an organic peroxide can be used.

Among chemical polymerizable initiators, the thiourea derivative functions as a reducing agent.

The thiourea derivative is not particularly limited. Examples of the thiourea derivative include ethylene thiourea, N-methylthiourea, N-ethylthiourea, N-propylthiourea, N-butylthiourea, N-laurylthiourea, N-phenylthiourea, N-cyclohexylthiourea, N,N-dimethylthiourea, N,N-diethylthiourea, N,N-dipropylthiourea, N,N-dibutylthiourea, N,N-dilaurylthiourea, N,N-diphenylthiourea, N,N-dicyclohexylthiourea, trimethylthiourea, tetramethylthiourea, N-acetylthiourea, N-benzoylthiourea, 1-allyl-3-(2-hydroxyethyl)-2-thiourea, 1-(2-tetrahydrofurfuryl)-2-thiourea, N-tert-butyl-N'-isopropylthiourea, 2-pyridylthiourea, and the like.

The above thiourea derivatives may be used alone or in combination. Among the above thiourea derivatives, N-benzoylthiourea is preferable in view of an improvement in curability of the dental composition.

The amount of the thiourea derivative in the dental composition is not particularly limited, and is preferably 0.1 percent by mass or greater and 5 percent by mass or less, more preferably 0.1 percent by mass or greater and 3 percent by mass or less, and yet more preferably 0.1 percent by mass or greater and 1 percent by mass or less. When the amount of the thiourea derivative in the dental composition is 0.1 percent by mass or greater, curability of the dental composition is further improved. When the amount of the thiourea derivative in the dental composition is 5 percent by mass or less, solubility of the thiourea derivative to (meth)acrylate in the dental composition is further improved.

Among chemical polymerization initiators, the vanadium compound functions as a reducing agent.

The vanadium compound is not particularly limited, and examples of the vanadium compound include oxovanadium oxalate, vanadyl acetylacetonate, vanadium acetylacetonate, vanadyl stearate, vanadium naphthenate, vanadium benzoylacetonate, and the like. The above vanadium compounds may be used alone or in combination. Among the above vanadium compounds, vanadyl acetylacetonate is preferable in view of curability of the dental composition.

The amount of the vanadium compound in the dental composition is not particularly limited, and is preferably 0.001 percent by mass or greater and 5 percent by mass or less, more preferably 0.0015 percent by mass or greater and 1 percent by mass or less, and yet more preferably 0.002 percent by mass or greater and 0.1 percent by mass or less. When the amount of the vanadium compound in the dental composition is 0.001 percent by mass or greater, curability of the dental composition is further improved. When the amount of the vanadium compound in the dental composition is 5 percent by mass or less, storage stability of the dental composition is further improved.

Among chemical polymerization initiators, the tertiary amine functions as a reducing agent.

The tertiary amine is not particularly limited, and examples of the tertiary amine include a tertiary aliphatic amine and a tertiary aromatic amine.

Examples of the tertiary aliphatic amine include N,N-dimethylaminoethyl methacrylate, triethanolamine, and the like.

Examples of the tertiary aromatic amine include alkyl p-dialkylaminobenzoate, 7-dimethylamino-4-methylcoumarin, N,N-dimethylaniline, N,N-dibenzylaniline, N,N-dimethyl-p-toluidine, N,N-diethyl-p-toluidine, N,N-bis(2-hydroxyethyl)-p-toluidine, N,N,2,4,6-pentamethylaniline, N,N,2,4-tetramethylaniline, N,N-diethyl-2,4,6-trimethylaniline, and the like.

Among the above tertiary amines, a tertiary aromatic amine is preferable, and alkyl p-dialkylaminobenzoate is more preferable.

Examples of the alkyl p-dialkylaminobenzoate include methyl p-dimethylaminobenzoate, ethyl p-dimethylaminobenzoate, propyl p-dimethylaminobenzoate, amyl p-dimethylaminobenzoate, isoamyl p-dimethylaminobenzoate, ethyl p-diethylaminobenzoate, propyl p-diethylaminobenzoate, and the like.

The above tertiary amines may be used alone or in combination.

Among chemical polymerization initiators, the organic peroxide functions as an oxidizing agent.

Examples of the organic peroxide include benzoyl peroxide, cumene hydroperoxide, t-butyl hydroperoxide, t-amyl hydroperoxide, 1,1,3,3-tetramethylbutyl hydroperoxide, 2,5-dimethyl-2,5-di(hydroperoxy)hexane, p-diisopropylbenzene monohydroperoxide, p-methane hydroperoxide, pinane hydroperoxide, and the like.

The above organic peroxides may be used alone or in combination. Among the above organic peroxides, cumene hydroperoxide is preferable in view of curability of the dental composition.

The amount of the organic peroxide in the dental composition is not particularly limited, and is preferably 0.01 percent by mass or greater and 10 percent by mass or less, more preferably 0.05 percent by mass or greater and 5 percent by mass or less, and yet more preferably 0.1 percent by mass or greater and 3 percent by mass or less. When the amount of the organic peroxide in the dental composition is 0.01 percent by mass or greater, curability of the dental composition is further improved. When the amount of the organic peroxide in the dental composition is 10 percent by mass or less, an operable time of the dental composition is prolonged.

The photopolymerization initiator is not particularly limited, and examples of the photopolymerization initiator include camphorquinone (CQ), ethyl 4-dimethylaminobenzoate (EPA), 2,4,6-trimethylbenzoyldiphenylphosphine oxide (TPO), phenyl bis(2,4,6-trimethylbenzoyl)phosphine oxide, benzyl ketal, deacetyl ketal, benzyl dimethyl ketal, benzyl diethyl ketal, benzyl bis(2-methoxyethyl) ketal, 4,4'-dimethyl(benzyldimethylketal), anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluoromethylthioxanthone, thioxanthone-10,10-dioxide, thioxanthone-10-oxide, benzoin methyl ether, benzoin ethyl ether, benzoin isopropyl ether, benzoin isobutyl ether, benzophenone, bis(4-dimethylaminophenyl)ketone, 4,4'-bis(diethylamino)benzophenone, and the like.

The above photopolymerization initiators may be used alone or in combination. Among the above photopolymerization initiators, camphorquinone (CQ), ethyl 4-dimethylaminobenzoate (EPA), and 2,4,6-trimethylbenzoyldiphenylphosphine oxide (TPO) are preferable in view of an improvement in curability of the dental composition.

The amount of the photopolymerization initiator in the dental composition is not particularly limited, and is preferably 0.01 percent by mass or greater and 5 percent by mass or less, more preferably 0.05 percent by mass or greater and 3 percent by mass or less, and yet more preferably 0.1 percent by mass or greater and 1 percent by mass or less. When the amount of the photopolymerization initiator in the dental composition is 0.01 percent by mass or greater, curability of the dental composition is improved. When the amount of the photopolymerization initiator in the dental composition is 5 percent by mass or less, storage stability of the dental composition is improved.

Examples of the polymerization inhibitor include dibutylhydroxytoluene (2,6-ditert-butyl-p-cresol), 6-tert-butyl-2,4-xylenol, 1,4-dihydroxybenzene, and the like.

The above polymerization inhibitors may be used alone or in combination. Among the above polymerization inhibitors, dibutylhydroxytoluene and 6-tert-butyl-2,4-xylenol are preferable in view of an improvement in curability of the dental composition.

The amount of the polymerization inhibitor in the dental composition is not particularly limited, and is preferably 0.001 percent by mass or greater and 5 percent by mass or less, more preferably 0.005 percent by mass or greater and 3 percent by mass or less, and yet more preferably 0.01 percent by mass or greater and 1 percent by mass or less. When the amount of the polymerization inhibitor in the dental composition is 0.001 percent by mass or greater, storage stability of the dental composition is improved. When the amount of the polymerization inhibitor in the dental composition is 5 percent by mass or less, curability of the dental composition is improved.

The filler refers to an inorganic filler. Examples of the filler include glass, glass compositions, dental glass compositions, inorganic crystals, and the like.

As described above, the dental composition of the present embodiment includes the dental glass composition of the present embodiment, and therefore the effect of the dental glass composition can be obtained. Specifically, the dental composition including the dental glass composition of the present embodiment can achieve both high radiopacity and storage stability in the presence of an acidic substance. In addition, the dental composition of the present embodiment exhibits favorable radiopacity, and therefore a restored site can be clearly visualized in diagnosis or examination by an X-ray image taken after treatment.

Use of the dental composition of the present embodiment is not particularly limited. Since the dental composition has high radiopacity, the dental composition can be used as a dental material requiring radiopacity. Examples of such dental material include a dental composite resin, a dental cement, a dental adhesive, a dental sealant, a dental primer, a dental coating material, a dental hard resin, a dental milling resin material, a temporary dental restorative material, a dental filler, a dentifrice, and the like.

### Examples

The present invention will be further described through Examples hereinafter. In the following description, a numerical value without a unit or "%" is based on mass unless otherwise specified.

### <Preparation of glass>

Each glass (each of Glass 1 to 8) was prepared according to the composition presented in Table 1. The glass was obtained as a bulk by placing a raw material batch, which was obtained by mixing raw materials, in a platinum crucible, setting the raw material batch to be retained in an electric furnace, which had been preheated at 1,400°C to 1,600°C, for 30 minutes to 60 minutes, and pouring the melt into a carbon mold. Alternatively, after melting in the same manner, the glass melt was dropped into a water tank to obtain granulated glass. The glass was pulverized in an alumina magnet motor to obtain a glass powder having a medium particle diameter of 20 µm to 300 µm.

In addition, the obtained bulk of glass was subjected to a heat treatment in which the bulk of glass was heated in the electric furnace from room temperature to 600 ° C at the heating rate of 20 ° C/min, the temperature was held at 600 ° C for 30 minutes, and the bulk body was slowly cooled to room temperature. The bulk of glass after the heat treatment was sliced to obtain a sliced piece.

### <Radiopacity>

Radiopacity (% relative to Al) of the obtained sliced piece of glass was measured. The radiopacity was measured by the radio-opacity test method for dental materials in accordance with ISO 4049:2019. Specifically, optical density or a grayscale of a test specimen (plate-shaped dental glass composition) having a thickness Ts (unit: mm) was measured. Next, a thickness Ta (unit: mm) of an aluminum plate having the same optical density or grayscale as the test specimen having the thickness Ts was determined. Then, ratio (unit: % relative to Al) of the thickness Ta to the thickness Ts was used as a value of radiopacity. The results of radiopacity are presented in Table 1.

The silica filler described below was a powdery sample. Therefore, a bulk could not be formed, and accurate radiopacity could not be measured.

**[Table 1]**

| Glass composition (mass%) | Glass 1 | Glass 2 | Glass 3 | Glass 4 | Glass 5 | Glass 6 | Glass 7 | Glass 8 | Silica filler |
|---|---|---|---|---|---|---|---|---|---|
| SiO₂ | 46.45 | 45.12 | 44.34 | 42.00 | 41.23 | 44.97 | 49.25 | 56.89 | 100.00 |
| B₂O₃ | 17.94 | 17.43 | 17.12 | 16.23 | 15.93 | 11.99 | 19.32 | 21.98 | |
| Al₂O₃ | 6.57 | 6.38 | 6.27 | 11.88 | 11.66 | 8.91 | 5.14 | 8.05 | |
| Na₂O | | | | 3.61 | | | | | |
| K₂O | | | | | 5.38 | | | | |
| CaO | | | | | | | 1.41 | | |
| F | | 2.85 | | | | 1.80 | | | |
| ZrO₂ | | | 4.55 | | | | | | |
| Cs₂O | 29.04 | 28.22 | 27.72 | 26.28 | 25.80 | | 24.88 | | |
| SrO | | | | | | | | 13.08 | |
| BaO | | | | | | 32.33 | | | |
| Total | 100.00 | 100.00 | 100.00 | 100.00 | 100.00 | 100.00 | 100.00 | 100.00 | 100.00 |
| Radiopacity of glass (% to AI) | 593 | 553 | 664 | 531 | 522 | 663 | 467 | 204 | Could not be measured |

### < Preparation of dental composition (test specimen)>

The glass or silica filler (RAR1000, manufactured by TATSUMORI LTD.) presented in Table 1 was mixed and stirred with 3-methacryloxypropyltrimethoxysilane (manufactured by Shin-Etsu Chemical Co., Ltd.) used as a silane coupling agent to perform a silane treatment, thereby obtaining an inorganic filler. In addition, a polymerizable monomer (polymerizable monomer having no acid functional group), an acidic substance (acidic group-containing polymerizable monomer), a polymerization initiator, and an inhibitor were kneaded at the blending ratio specified in Table 2, thereby preparing an organic component.

Each of the obtained inorganic fillers, aerosil (manufactured by NIPPON AEROSIL CO., LTD.), and the organic component presented in Table 2 were mixed at the blending ratio presented in Table 3, thereby preparing a dental composition (test specimen). In Table 3, Glass 1 to 5, 7, and 8 were mixed at the blending ratio 1, Glass 6 was mixed at the blending ratio 2, and the silica filler was mixed at the blending ratio 3.

**[Table 2]**

| | Substance | Blending ratio (mass%) |
|---|---|---|
| Polymerizable monomer | TEGDMA | 48 |
| | UDMA | 34 |
| Acidic substance | MDP | 16 |
| Polymerization initiator/ inhibitor | CQ | 0.5 |
| | EPA | 1.0 |
| | IA | 0.2 |
| | BHT | 0.3 |

The substances corresponding to the abbreviations of the substances in Table 2 are as follows.
TEGDMA: triethylene glycol dimethacrylate
UDMA: urethane dimethacrylate
MDP: 10-methacryloyloxydecyl dihydrogen phosphate
CQ: camphorquinone
EPA: ethyl 4-dimethylaminobenzoate
IA: 6-tert-butyl-2,4-xylenol
BHT: dibutylhydroxytoluene

**[Table 3]**

| Blending ratio (mass%) | Blending ratio 1 | Blending ratio 2 | Blending ratio 3 |
|---|---|---|---|
| Glass | Glass 1 to 5, 7, 8 | Glass 6 | Silica filler |
| Organic component | 43 | 32 | 34 |
| Aerosil | 3 | 2 | 2 |
| Inorganic filler | 54 | 66 | 64 |

### <Storage stability test>

A storage stability test was performed on each of the obtained dental compositions (test specimens) to determine storage stability. A filling syringe was filled with each obtained dental composition (test specimen), and was stored in a thermo-hydrostat at 37 ° C and 90%RH. Separately, water (0.8%) in an amount equivalent to 3-month storage at 37 ° C and 90%RH was added to the paste immediately after preparation. The dental composition (test specimen) immediately after preparation or after adding water was irradiated with light to polymerize the dental composition, thereby obtaining a cured product of the dental composition (test specimen) (dental composition cured product).

### <Evaluation criteria for storage stability>

The storage stability of the dental composition (test specimen) immediately after preparation or after the storage stability test was determined by the following two methods. The viscosity of the dental composition (test specimen) was evaluated by a rheometer. In the case where the viscosity (mPa·s) of the dental composition (test specimen) after the storage stability test was increased by 10% or more compared to the viscosity of the dental composition (test specimen) immediately after the preparation, the storage stability was evaluated as being poor. In addition, in the case where formation of the acidic substance (MDP) and the salts derived from the glass melt (detected as needle-like substances in the SEM image) were confirmed by observation of the cured body (dental composition cured body), which was cured by photopolymerization, under a scanning electron microscope (SEM), the storage stability was evaluated as being poor. Further, in the case where the defects were not observed in both the SEM observation and the evaluation by the rheometer, the storage stability was determined as being good. In the case where the defect was observed in either the SEM observation or the evaluation by the rheometer, the storage stability was determined as being poor. The evaluation results of the storage stability test are presented in Table 4.

### <Measurement of radiopacity of dental composition cured product>

Radiopacity (% relative to Al) was measured on the cured product (dental composition cured product) obtained by curing the obtained dental composition (test specimen) through photopolymerization. The radiopacity was measured by the radio-opacity test method for dental materials in accordance with ISO 4049:2019. Specifically, the optical density or grayscale of the test specimen (dental composition cured product) having a thickness Ts (unit: mm) was measured. Next, the thickness Ta (unit: mm) of an aluminum plate having the same optical density or grayscale as the optical density or grayscale of the test specimen having the thickness of Ts was measured. Then, the ratio (unit: % relative to Al) of the thickness Ta to the thickness Ts was used as the value of radiopacity. In the case where the radiopacity (unit: % relative to Al) of the cured product of the dental composition was 200 or less, the radiopacity was determined as being not acceptable.

**[Table 4]**

| Example/ Comparative Example | Example | | | Comparative Example | | | | | |
|---|---|---|---|---|---|---|---|---|---|
| | 1 | 2 | 3 | 1 | 2 | 3 | 4 | 5 | 6 |
| Used glass | Glass 1 | Glass 2 | Glass 3 | Glass 4 | Glass 5 | Glass 6 | Glass 7 | Glass 8 | Silica filler |
| Immediately after production | Good | Good | Good | Poor | Poor | Poor | Good | Good | Good |
| Water addition conditions (equivalent to 3-month storage at 37°C and 90RH) | Good | Good | Good | - | Poor | Poor | Poor | Poor | Good |
| Storage stability test | Good | Good | Good | Poor | Poor | Poor | Poor | Poor | Good |
| Radiopacity of dental composition cured product (% relative to AI) | 285 | 288 | 266 | 332 | 237 | 300 | 211 | 124 | 85 |

According to Tables 1 to 4, the dental composition, each of which satisfield the conditions of including silicon (Si) and cesium (Cs) and being substantially free from sodium (Na), potassium (K), and one or more alkaline earth metals, were evaluated as being good in radiopacity (Examples 1 to 3).

Conversely, the dental compositions including the glass or silica fiiler, each of which did not satisfy the conditions of including silicon (Si) and cesium (Cs) and being substantially free from sodium (Na), potassium (K), and one or more alkaline earth metals, were poor in the storage stability, the radiopacity, or both the storage stability and the radiopacity (Comparative Examples 1 to 6).

Although the embodiments of the present invention have been described above, the present invention is not limited to the specific embodiments, and various modifications and changes can be made within the scope of the invention described in the claims.

The present application claims priority based on Japanese Patent Application No. 2023-140970 filed on August 31, 2023, the entire contents of which are incorporated herein by reference.

## Claims

1. A dental glass composition comprising:
silicon (Si); and
cesium (Cs),
wherein the dental glass composition is substantially free from sodium (Na), potassium (K), and one or more alkaline earth metals.

2. A dental glass composition comprising:
silicon (Si); and
cesium (Cs),
wherein the dental glass composition is substantially free from sodium (Na), potassium (K), calcium (Ca), strontium (Sr), and barium (Ba).

3. A dental composition comprising:
the dental glass composition according to claim 1 or 2; and
an acidic group-containing polymerizable monomer.
